# FASCICULE DE BREVET EUROPEEN

(11) **EP 1 151 852 B1**
(45) Date de publication et mention de la délivrance du brevet: **24.11.2004**
(21) Numéro de dépôt: 00401191.2
(22) Date de dépôt: 28.04.2000
(51) Int. Cl.: B31F 1/07

(54) **Dispositif et procédé pour le serrage de papier ou non tissé, et article fabriqué**
Vorrichtung und Verfahren zum Prägen von Papier oder Vliesstoff, sowie das hergestellte Produkt
Device and method for embossing of paper or nonwovens, and product obtained

(43) Date de publication de la demande: 07.11.2001
(73) Titulaire: Georgia-Pacific France, 68320 Kunheim (FR)
(72) Inventeur: Levebvre du Grosriez, Carol, 68127 Oberhergheim (FR)
(74) Mandataire: Cortier, Sophie

(56) Documents cités:
- GB-A- 1 474 101
- US-A- 5 122 221

## Description

L'invention concerne un dispositif pour le serrage d'au moins une feuille de papier ou nontissé et trouve application dans la transformation de la feuille en produits finis après sa fabrication par voie humide ou voie sèche.

L'invention concerne plus particulièrement un dispositif pour le serrage d'au moins une feuille de papier ou nontissé, comprenant deux cylindres sensiblement tangents l'un à l'autre, entre lesquels passent la feuille. Dans ce dispositif, au moins un des cylindres comporte sur sa surface dans une zone destinée à être en contact avec la feuille, une bande de serrage comprenant des premiers éléments en relief. L'interaction des deux cylindres avec ou sans la feuille définit une surface active au cours de la rotation des cylindres. Cette surface active sera plus précisément déterminée dans la description qui suit.

Le document US 5 122 221 A décrit un tel dispositif.

On entend par papier tout produit à base de fibres papetières tel que la ouate de cellulose, des produits papetiers absorbants obtenus par voie humide ou par voie sèche. Dans ces derniers, les fibres papetières sont liées par un liant thermoplastique tel qu'un latex ou des fibres thermofusibles. Certains produits papetiers peuvent également comporter des fibres cellulosiques autres que papetières, et parfois même, partiellement, des fibres synthétiques ou artificielles.

Les produits nontissés sont également visés par l'invention.

L'invention est plus particulièrement destinée à la fabrication d'articles en ouate de cellulose, et notamment de serviettes en papier, de mouchoirs, de papiers d'essuyage (essuie-tout) ou de papiers toilette.

Ces articles sont généralement réalisés à partir d'une ou plusieurs feuilles de papier (ou plis) superposées, qui subissent une opération de serrage. Celle-ci peut avoir plusieurs finalités.

En effet, dans la suite du texte, le serrage d'une ou plusieurs feuilles entre deux cylindres (de préférence contrarotatifs) du dispositif, en fonction des conditions opératoires notamment de pression et de la nature des cylindres, peut conduire à :
la simple association des feuilles entre elles, avec ou sans apport d'additif,
un dépôt d'additif sur la surface d'une feuille, par exemple pour l'application de colle, d'encre ou de lotion, ou
un marquage d'une ou plusieurs feuilles passant dans le dispositif.

On entend par simple association, le fait de solidariser plusieurs feuilles entre-elles par simple pression, avec ou sans adhésif. Dans le cas d'un encollage, le dispositif comprend un cylindre gravé et un contre-cylindre qui est le cylindre applicateur portant le film de colle.

On entend par marquage tout type de déformation de la feuille de papier ou nontissé, susceptible d'être obtenue par le passage de la feuille entre deux cylindres dont l'un au moins est pourvu d'une bande de serrage. En serrant la feuille de papier contre la surface d'un contre-cylindre, la bande de serrage provoque le marquage de la feuille, soit par compression des fibres du papier (marquage au sens strict), soit par déformation en relief (gaufrage), selon des motifs correspondant à des éléments en relief disposés sur la bande de serrage.

Selon les utilisations de ce dispositif, la bande de serrage formera sur le papier ou le nontissé un motif permanent ou non. En effet, au moment même où les cylindres sont en contact l'un avec l'autre par l'intermédiaire de la feuille de papier ou nontissé, la pression quand elle s'exerce, induit un marquage au moyen de la bande de serrage. L'intensité de cette pression (si elle est relativement faible dans le cas d'une association de deux feuilles par exemple) peut être telle que le marquage est léger et n'est pas permanent. Généralement, dans ce cas, aucun marquage n'est visible sur le produit fini. Avec une pression élevée, le marquage devient permanent et visible sur le produit fini.

La bande de serrage comprend des premiers éléments en relief qui sont gravés sur sa surface cylindrique. Ces éléments peuvent être disposés de manière à former, autour du cylindre, une ou plusieurs zones en relief continues ou non. Ils sont du type géométrique ou non. Ils peuvent être constitués d'éléments unitaires du type tirets, points, lignes continues ou discontinues, ou autres. On peut également envisager de réaliser des motifs par manque d'éléments en relief ou picots. A cette fin, des zones sans picots de la forme du motif souhaité sur le papier, sont prévues sur une surface contenant une répartition régulière de picots.

En fonction notamment de la nature du contre-cylindre et de la pression exercée par les cylindres sur la feuille de papier, on peut obtenir des marquages de natures différentes.

Ainsi, le revêtement du contre-cylindre peut être constitué d'un matériau relativement souple par rapport au matériau du revêtement du cylindre portant la bande de serrage. Le contre-cylindre peut encore être réalisé dans un matériau similaire à celui du cylindre portant la bande de serrage. La surface cylindrique externe du contre-cylindre peut être lisse; elle peut être pourvue d'éléments en creux complémentaires des éléments en relief de la bande de serrage ou elle peut être pourvue d'éléments en relief identiques à ceux de la bande de serrage de manière que la feuille de papier soit serrée sous une certaine pression entre deux éléments en relief face à face.

En fonction de ces divers paramètres, la nature du marquage pourra avoir diverses conséquences telles que notamment une augmentation de l'épaisseur du papier (gaufrage), une augmentation du pouvoir absorbant du papier, l'accrochage des différents plis formant la feuille de papier, un marquage particulièrement esthétique ou encore une diminution de l'épaisseur du papier (marquage au sens strict) dans les zones de motif.

Dans tous les cas, le serrage de la feuille de papier s'effectue entre deux cylindres d'axes sensiblement parallèles, amenés radialement en appui l'un contre l'autre, et qui sont en rotation lorsque la feuille de papier défile entre les deux cylindres.

Le serrage du papier se fait donc au niveau de la zone de contact des deux cylindres.

Cette zone de contact, qui, en théorie, est une ligne parallèle aux axes de rotation des deux cylindres, est en réalité une zone qui présente une certaine largeur selon la circonférence des cylindres (diamètre) et leur laize, la pression exercée entre les cylindres, la nature et les caractéristiques (épaisseur, dureté...) des revêtements des cylindres et les caractéristiques (épaisseur) du papier ou nontissé qui est serré. Cette zone présente ainsi la forme d'une bande de contact qui s'étend axialement le long d'une génératrice principale de contact. La forme de cette zone n'est pas nécessairement homogène sur la laize, elle peut être rectangulaire, creusée au centre, bombée au centre, etc. Cette zone de pincement en forme de bande de contact des deux cylindres est généralement désignée par le terme anglais "nip".

Il est à noter que lorsque l'épaisseur de la feuille de papier ou nontissé est supérieure à l'intervalle existant entre les deux cylindres, le « nip » ou zone de pincement peut également exister entre les cylindres, le contact se faisant par l'intermédiaire de la feuille.

Le document GB 1 474 101 A montre différents types de marquage qui peuvent être réalisés sur une feuille de papier ou nontissé.

Au cours de leur rotation, les deux cylindres interagissent entre-eux avec ou sans la feuille, et déterminent une surface active engendrée notamment par les éléments en relief disposés en surface des cylindres. Plus précisément, la surface active correspondant à la bande de serrage comprend la surface des éléments en relief qui se trouvent en contact avec la contrepartie par l'intermédiaire de la feuille de papier et, le cas échéant, en particulier lorsque la contrepartie est du type caoutchouc, la surface d'une partie des zones situées entre les éléments en relief, zones qui par la déformation de la contrepartie et du papier, peuvent également être en contact avec la contrepartie par l'intermédiaire de la feuille de papier. Dans ce dernier cas, la pression exercée varie entre le sommet des éléments en relief et les zones de contact entre les éléments en relief.

A chaque nip sera associée une surface active que l'on qualifiera de surface active instantanée.

Ainsi, les surfaces actives instantanées successives par lesquelles les deux cylindres coopèrent l'un avec l'autre, varient en fonction des éléments en relief de la bande de serrage mais aussi en fonction de la superficie de la zone de contact.

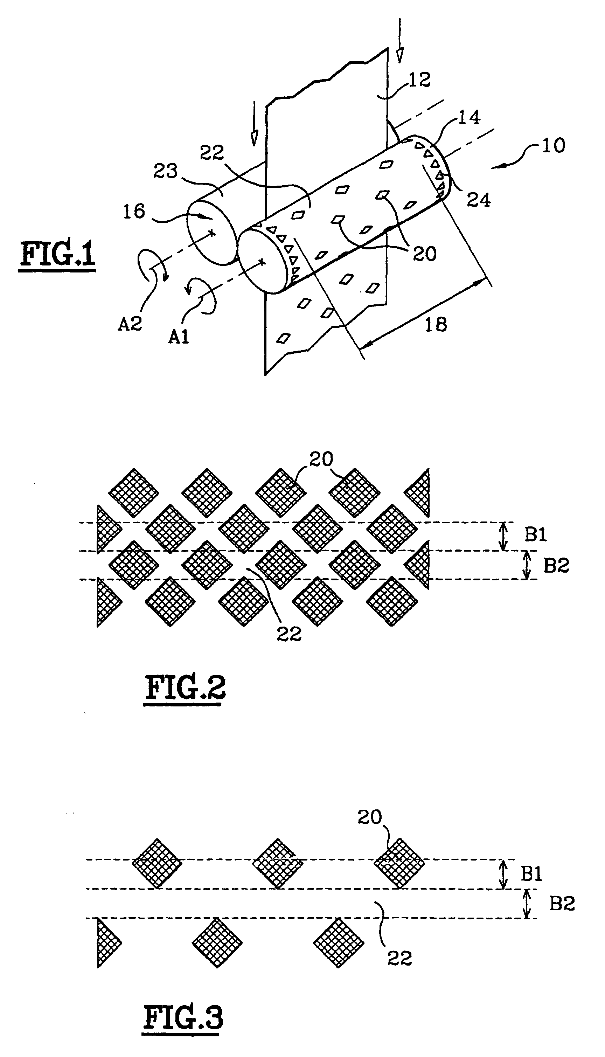

On a illustré par les figures 2 et 3 deux exemples de disposition possible des éléments en relief pour une bande de serrage.

Pour les dispositions illustrées aux figures 2 et 3, on a délimité deux bandes B1 et B2 qui représentent la zone de contact des deux cylindres pour deux positions angulaires différentes de ces deux cylindres.

Dans l'exemple de la figure 2, si l'on considère les zones de contact B1, B2 ; on constate que la surface active instantanée de la bande B1 est identique à celle de la bande B2.

Dans l'exemple de la figure 3, les deux bandes B1 et B2 présentent des surfaces actives instantanées successives très différentes étant donné que, contrairement à la bande B1, la surface des éléments en relief de la bande B2 est nulle.

Dans la position angulaire des cylindres correspondant à la bande B2, dans le cas d'un travail en pression des cylindres, il y a une interaction entre ces deux cylindres en contact mais elle est très différente de l'interaction de ces mêmes cylindres dans la position angulaire correspondant à la bande B1. Pour la disposition des éléments en relief selon la figure 3, les efforts encaissés par les cylindres varient donc fortement dans le temps au cours de la rotation des cylindres.

Une telle variation des efforts d'interaction entre les deux cylindres qui permettent ici le serrage, est très défavorable à la résistance et au vieillissement des cylindres et de toute l'installation en général (création de vibrations). Le problème se pose dans de nombreux dispositifs permettant le serrage d'une feuille de papier ou nontissé entre deux cylindres dont l'un au moins a une surface gravée.

L'invention a donc pour but de proposer une amélioration des dispositifs de serrage d'une feuille de papier ou nontissé qui permet d'assurer une durée de vie plus importante des cylindres et de l'ensemble de l'installation. L'invention a également pour but de permettre la réalisation sur la feuille de motifs extrêmement variés, en se dégageant de toute contrainte quant à la régularité des efforts d'interaction entre les cylindres induits par les éléments en relief, qui réduit le choix des motifs.

Ainsi, des motifs de densité, répartition et formes différentes peuvent être maintenant choisis.

Dans ce but, l'invention propose un dispositif de serrage d'une feuille de papier ou nontissé, du type décrit précédemment, caractérisé en ce que l'un au moins des cylindres comporte sur sa surface dans une zone destinée à être en contact avec l'autre cylindre par l'intermédiaire ou non de la feuille, une bande de compensation de charge comprenant des seconds éléments en relief et choisie de façon que la variation des surfaces actives instantanées successives correspondant à la bande de serrage et à la bande de compensation de charge, soit inférieure à la variation des surfaces actives instantanées successives correspondant à la seule bande de serrage.

Selon d'autres caractéristiques de l'invention, prises seules ou en combinaison :
- la variation des surfaces actives instantanées successives correspondant à la bande de serrage et à la bande de compensation de charge est sensiblement nulle ;
- la largeur de la surface active correspondant à la seule bande de compensation de charge varie, le long de la circonférence du cylindre sur lequel cette bande a été créée, en fonction de la surface active instantanée correspondant à la seule bande de serrage;
- les deux cylindres sont contrarotatifs ;
- les premiers éléments en relief de la bande de serrage sont identiques ou différents des seconds éléments en relief de la bande de compensation de charge ;
- la bande de compensation de charge est agencée sur un cylindre en dehors de la bande de serrage ;
- les seconds éléments en relief de la bande de compensation de charge sont différents des premiers éléments en relief de la bande de serrage et la bande de compensation de charge chevauche au moins partiellement la bande de serrage ;
- au moins un des cylindres porte au moins une bande de serrage et au moins une bande de compensation de charge ;
- la hauteur des éléments en relief de la bande de compensation de charge est sensiblement identique à la hauteur des éléments en relief de la bande de serrage ;
- la hauteur des éléments en relief de la bande de compensation de charge est supérieure à la hauteur des éléments en relief de la bande de serrage ;
- la hauteur des éléments en relief de la bande de compensation de charge est inférieure à la hauteur des éléments en relief de la bande de serrage ;
- la bande de compensation de charge comporte une zone de roulement qui détermine une largeur minimale de la surface active de la bande de compensation de charge ;
- la zone de roulement de la bande de compensation de charge est agencée radialement au même niveau que le reste de la bande de compensation de charge ;
- la zone de roulement de la bande de compensation de charge est agencée radialement vers l'extérieur par rapport au reste de la bande de compensation de charge ;
- la zone de roulement de la bande de compensation de charge est agencée radialement vers l'intérieur par rapport au reste de la bande de compensation de charge ;
- plusieurs bandes de compensation de charge sont prévues sur le même cylindre ;
- chacune des bandes de compensation de charge comporte une zone de roulement.

L'invention concerne aussi un cylindre pour le serrage d'une feuille de papier ou nontissé, caractérisé en ce qu'il comporte une bande de compensation de charge selon l'une quelconque des caractéristiques ci-dessus.

L'invention concerne également une installation comprenant au moins un dispositif selon l'une quelconque des caractéristiques précitées.

L'invention concerne encore un procédé d'association d'au moins deux feuilles de papier ou nontissé, caractérisé en ce qu'il comporte la mise en oeuvre d'un dispositif comportant l'une quelconque de ces caractéristiques, dans lequel les feuilles sont serrées entre les deux cylindres au niveau de la bande de serrage avec ou sans apport d'adhésif.

L'invention vise également un procédé de marquage d'au moins une feuille de papier ou nontissé, caractérisé en ce qu'il comporte la mise en oeuvre d'un dispositif comportant ces mêmes caractéristiques, dans lequel la bande de serrage réalise sur le papier ou nontissé un motif par déformation de la feuille qui est serrée entre les deux cylindres.

L'invention englobe également tout procédé mettant en oeuvre le dispositif selon l'invention pour appliquer des additifs sur une feuille de papier ou nontissé.

L'invention concerne aussi un procédé de fabrication d'articles en ouate de cellulose comportant au moins deux feuilles, caractérisé en ce que les feuilles sont rendues solidaires les unes aux autres par un procédé de marquage, gaufrage ou association.

D'autres caractéristiques et avantages de l'invention apparaîtront plus clairement à la lecture de la description détaillée qui suit et des dessins annexés dans lesquels :
- la figure 1 est une vue schématique en perspective illustrant le principe d'un dispositif de serrage d'une feuille de papier par passage de la feuille entre deux cylindres ;
- les figures 2 et 3 sont deux exemples de disposition d'éléments en relief pour une bande de serrage ;
- les figures 4, 5 et 6 sont des représentations schématiques illustrant les surfaces actives d'une bande de serrage et d'au moins une bande de compensation de charge prévues sur un cylindre d'un dispositif selon l'invention ;
- la figure 7 est une vue similaire à celle des figures 4 à 6 dans lesquelles les bandes de compensation de charge du dispositif comportent une zone de roulement ;
- les figures 8 à 10 sont des diagrammes illustrant de manière schématique différentes positions des éléments en relief de la bande de serrage par rapport à ceux de la bande de compensation de charge ;
- la figure 11 est un diagramme similaire à celui des figures 8 à 10 illustrant un exemple d'une position relative possible, selon la direction radiale, de la zone de roulement d'une bande de compensation de charge par rapport à cette bande de compensation de charge ;
- les figures 12 et 13 sont des représentations schématiques illustrant les surfaces actives d'une bande de serrage, de deux bandes de compensation de charge et de deux zones de roulement prévues sur un cylindre d'un dispositif destiné au marquage des bordures de produits en formats ; et
- les figures 15 à 17 illustrent des bandes de compensation de charge partielles susceptibles d'être utilisées en remplacement de la bande de compensation de charge théorique représentée à la figure 14.

Dans la description qui suit, un exemple de dispositif de serrage illustre plus précisément l'invention. Ce dispositif entraîne la déformation d'une feuille de papier, en l'occurrence son marquage.

On a représenté sur la figure 1 un dispositif 10 dans lequel une feuille de papier 12 est marquée par passage entre deux cylindres 14, 16 d'axes parallèles A1 et A2 qui tournent de façon synchrone en sens inverse l'un par rapport à l'autre autour de leur axe respectif.

La feuille de papier 12 peut être remplacée par deux feuilles de papier, et leur serrage aura alors non seulement pour but de les marquer mais également de les rendre solidaires. Le marquage (dans le cas du gaufrage) peut aussi permettre d'augmenter le pouvoir absorbant du papier. Dans tous les cas, on cherchera à obtenir un marquage le plus esthétique possible.

Dans l'exemple représenté, la surface cylindrique 22 de l'un des cylindres 14 est gravée de manière à présenter une bande de serrage 18 qui comporte une pluralité d'éléments en relief 20.

Dans cet exemple de réalisation, le second cylindre 16, dit contre-cylindre, comporte une surface cylindrique 23 lisse.

Les deux cylindres 14 et 16 coopèrent l'un avec l'autre par une zone de contact de leur surface cylindrique 22, 23. Cette zone de contact, désignée également par le terme "nip", n'est pas limitée à une droite génératrice mais se présente sous la forme d'une bande axiale possédant une certaine dimension selon la direction circonférentielle des cylindres 14, 16 qui est aussi la direction de défilement de la feuille de papier 12.

Pour chaque position angulaire des cylindres 14, 16, correspondant par ailleurs à une position particulière de la feuille de papier selon la direction de défilement, les éléments en relief 20 de la bande de serrage 18 qui sont situés dans la zone de contact ou "nip", sont donc au contact de la feuille de papier 12 et provoquent un serrage de portions de celle-ci contre la surface cylindrique 23 du contre-cylindre 16.

La dimension de cette zone de contact selon la direction circonférentielle varie notamment en fonction de la nature des matériaux constitutifs des cylindres 14, 16, de leur diamètre et de leur écartement.

Conformément aux enseignements de l'invention, le cylindre gravé 14 comporte aussi, sur sa surface cylindrique 22, deux bandes de compensation de charge 24 qui sont agencées ici chacune à une extrémité axiale du cylindre gravé 14, de part et d'autre de la bande de serrage 18, à l'extérieur de la zone du cylindre 14 qui est en regard de la feuille de papier 12.

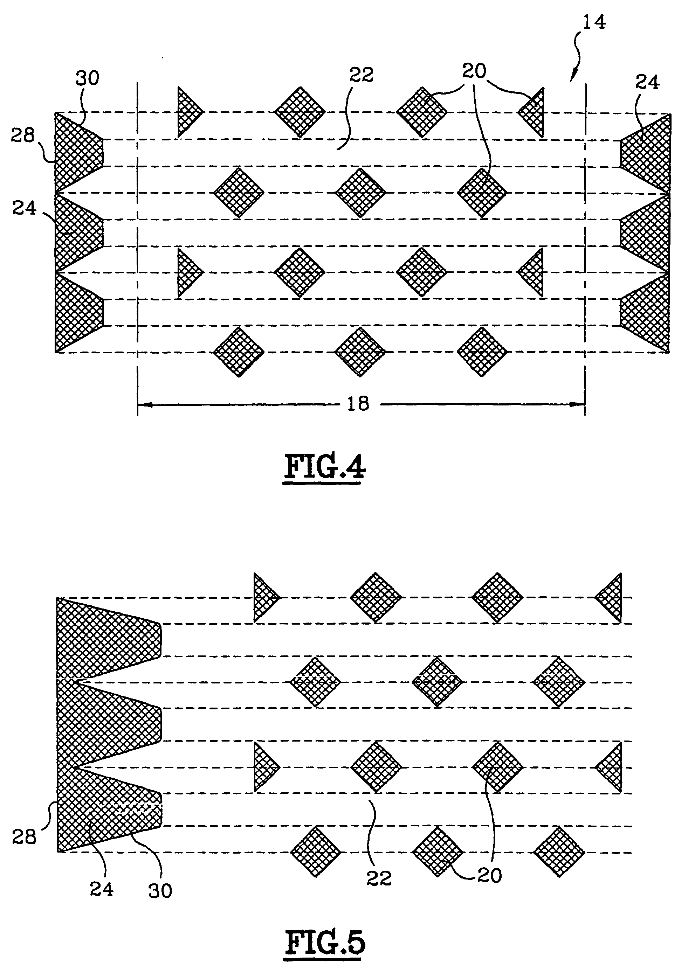

Pour les figures 4, 5 et 6, les surfaces actives correspondent uniquement aux éléments en relief.

La figure 4 représente en développé les éléments en relief de la surface du cylindre 14.

Dans cet exemple de réalisation, les éléments en relief 20 sont formés de losanges ou de triangles. Ce sont des formes géométriques simples mais l'invention peut bien entendu être réalisée pour des motifs de forme plus complexe et plus ornementale. De plus, les éléments en relief 20 peuvent par exemple être réalisés sous la forme d'une série de points, ce qui diminue la surface active de la bande de serrage 18 qui est véritablement destinée à venir au contact du contre-cylindre 16.

Il est à noter que, pour la bande de serrage, certaines dispositions d'éléments en relief composées uniquement de points, peuvent être plus denses que d'autres dispositions d'éléments formés de lignes et donc présenter une surface active plus importante. Une même disposition composée de lignes transformées en points peut permettre de diminuer la surface active de la bande de serrage qui est destinée à venir au contact du contre-cylindre par l'intermédiaire de la feuille de papier.

On qualifie de bande de compensation de charge, une zone d'éléments en relief prévue pour compenser les variations d'efforts entraînés par la surface active de la bande de serrage ; cette zone peut être constituée d'éléments en relief indépendants (petits éléments de type picot) disposés de manière discontinue ou encore être sous la forme d'une bande continue.

Dans l'exemple de réalisation de la figure 4, une bande de compensation de charge 24 est agencée de chaque côté de la bande de marquage 18 mais, ainsi que cela est représenté sur la figure 5, on peut prévoir de ne disposer qu'une seule bande de compensation de charge 24 à l'une des extrémités axiales du cylindre 14, cette bande étant alors de plus grande largeur.

Chaque bande de compensation de charge 24 est donc destinée à venir en appui contre la surface cylindrique 23 du contre-cylindre 16, sans l'intermédiaire de la feuille de papier 12 dans le mode de réalisation illustré à la figure 1 et par l'intermédiaire de la feuille de papier dans d'autres cas comme cela est développé plus loin dans la description. Pour chaque position angulaire des cylindres 14, 16, la surface active instantanée de la bande de compensation de charge 24 correspond à la partie de la bande 24 qui est comprise dans la zone de contact des deux cylindres ou "nip".

Comme on peut le voir sur les figures 4 à 6, la surface active de la bande de compensation de charge 24 est évolutive. Elle varie en sens inverse de la surface active de la bande de serrage 18 pour la même zone de contact.

Dans cet exemple, la variation des surfaces actives instantanées successives correspondant à la bande de serrage 18 et à la bande de compensation de charge 24 est sensiblement nulle au cours de la rotation complète des cylindres.

Ainsi, l'effort d'interaction entre les deux cylindres 14, 16 est sensiblement constant, ce qui est favorable à la bonne tenue dans le temps des cylindres et de l'ensemble du dispositif.

Dans les exemples de réalisation de l'invention représentés aux figures 4 à 6, la bande de compensation de charge 24 comporte un premier bord 28 qui, en développé, est rectiligne, et un second bord non rectiligne 30 dont le profil détermine la largeur de la bande de compensation de charge 24. Toutefois, on peut aussi prévoir que la bande de compensation de charge 24 soit délimitée par deux bords non rectilignes.

Pour définir la forme de la bande de compensation de charge, la circonférence du cylindre est analysée par zones de mesures successives. Ces zones pourront correspondre en valeur à un « nip ». Mais, plus la zone de mesure est fine, plus la bande de compensation de charge gagne en précision et moins les variations de charge sont importantes. De ce fait, la zone de mesure est de préférence inférieure au « nip ».

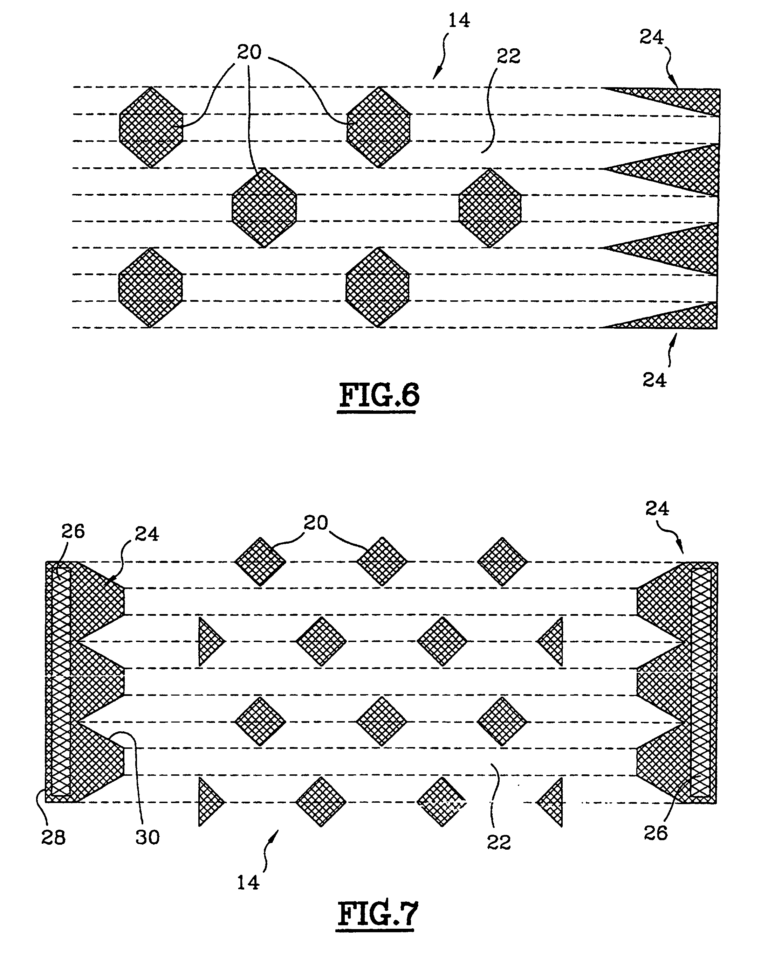

Comme on peut le voir sur la figure 6, la largeur de la bande de compensation de charge 24 peut, à certains endroits, être nulle. Cela se produit lorsque, dans une zone de contact considérée, la surface active de la bande de serrage 18 atteint une valeur maximale.

Toutefois, pour améliorer la tenue dans le temps des revêtements des cylindres 14, 16, il est souhaitable d'éviter de telles zones où la bande de compensation 24 est interrompue. En effet, la discontinuité locale d'efforts due à l'interruption de la bande de compensation de charge peut être, à la longue, nuisible au revêtement du cylindre.

Comme cela est représenté à la figure 7, on peut aussi prévoir que la bande de compensation de charge 24 comporte une zone de roulement 26 de largeur constante qui constitue en quelque sorte une surface minimale d'interaction entre les deux cylindres 14, 16.

Cette zone de roulement 26, de préférence adjacente au reste de la bande de compensation de charge 24, s'étend ainsi de manière continue autour de l'axe du cylindre.

Lorsque la bande de compensation de charge 24 présente deux bords non rectilignes, la zone de roulement 26 est par exemple prévue au centre de cette bande 24.

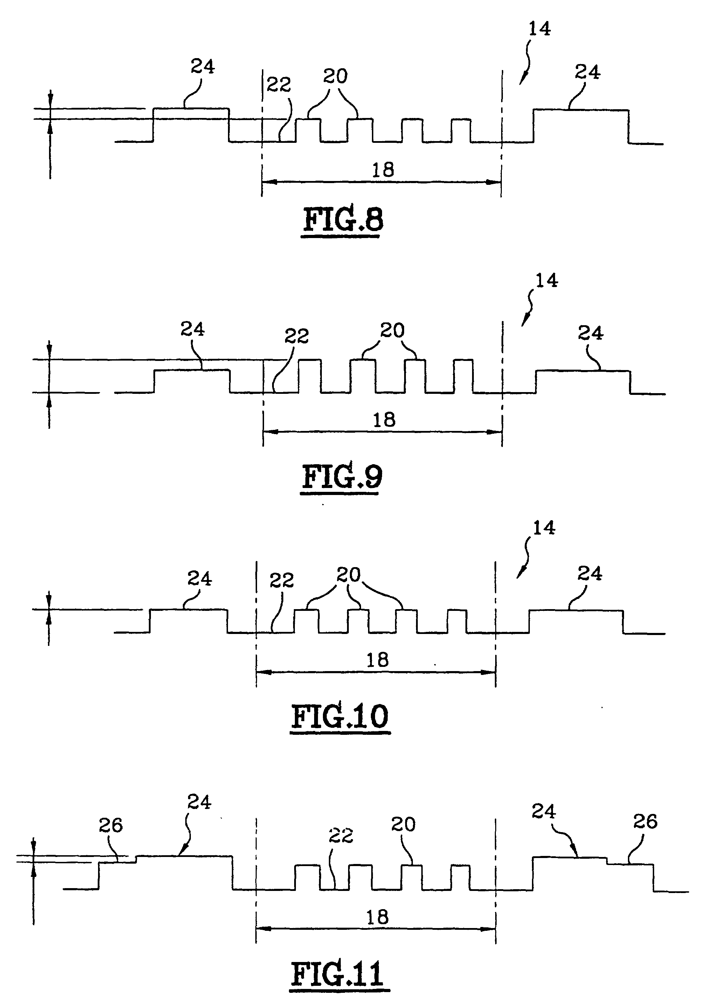

On a représenté sur les figures 8 à 10 trois diagrammes illustrant de manière schématique la position des éléments en relief de la bande de serrage 18 par rapport à ceux de la bande de compensation de charge 24.

Dans l'exemple de réalisation représenté sur la figure 8, la hauteur des éléments en relief de la bande de compensation de charge 24 est supérieure à la hauteur des éléments en relief 20 de la bande de serrage 18. Au contraire, dans la figure 9, la hauteur des éléments en relief 20 de la bande de compensation de charge 24 est inférieure à la hauteur des éléments en relief 20 de la bande de serrage 18, tandis que, à la figure 10, on a représenté le cas où la hauteur des éléments en relief de la bande de compensation de charge 24 est sensiblement identique à la hauteur des éléments en relief de la bande de serrage 18.

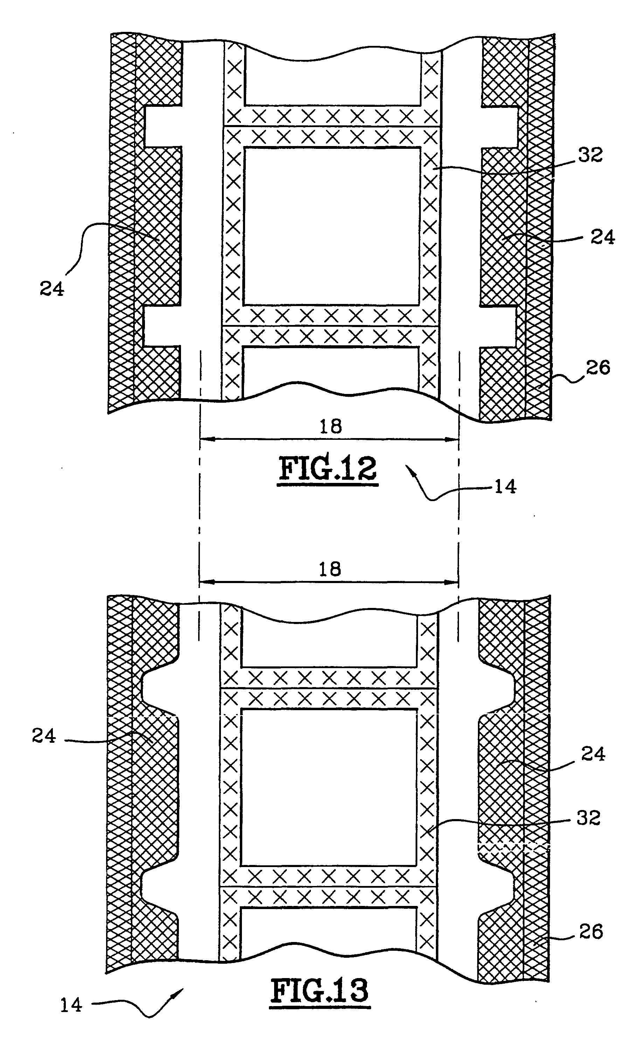

On a représenté sur la figure 11 un diagramme qui illustre le cas dans lequel la zone de roulement 26 est agencée radialement plus vers l'intérieur que le reste de la bande de compensation de charge 24. Toutefois, la zone de roulement 26 peut tout aussi bien être agencée radialement au même niveau ou plus vers l'extérieur que le reste de la bande de compensation de charge 24.

Dans les exemples de réalisation des figures 4 à 11, la bande de compensation de charge 24 et la bande de serrage 18 sont toutes les deux portées par le même cylindre 14. Cela est particulièrement avantageux lorsque le second cylindre 16 est lisse car un seul des deux cylindres doit être gravé, ce qui permet de réaliser l'invention sans surcoût notable. Toutefois, rien ne s'oppose à ce que les deux bandes 18 et 24 soient portées chacune par un des cylindres 14, 16. Dans le cas où la bande de compensation de charge n'est pas située sur le cylindre comportant les éléments en relief de la bande de serrage, il est nécessaire de prévoir un bon réglage angulaire entre les deux cylindres pour pouvoir caler la bande de compensation de charge avec la répartition des éléments en relief de la bande de serrage. De même, la zone de roulement peut être portée par le second cylindre 16.

Les figures 12 et 13 illustrent des modes de réalisation particuliers du dispositif selon l'invention, destiné au marquage des bordures de produits en papier absorbant qui sont découpés en formats. Ces produits sont par exemple des serviettes de table ou des mouchoirs en papier, jetables.

La bande de serrage 18 du cylindre 14 comprend des zones de bordure 32 comportant des éléments en relief destinés à marquer des produits en papier absorbant constitués d'au moins deux plis et à lier ces deux plis au niveau des bordures ou zones périphériques des produits en formats.

Ici, une bande de compensation de charge 24 est agencée de part et d'autre de la bande de serrage. Les figures 12 et 13 illustrent deux formes géométriques possibles pour la bande de compensation de charge 24.

La bande de compensation de charge 24 comporte également une zone de roulement 26.

Il a été vu que, dans les modes de réalisation envisagés précédemment, la bande de compensation de charge 24 est agencée en dehors de la bande de serrage 18 du cylindre 14 en regard de laquelle passe la feuille de papier 12 si bien que la bande de compensation de charge 24 ne laisse pas subsister de traces sur le papier.

Toutefois, dans certaines applications, la feuille de papier 12 est destinée à la fabrication d'articles en papier qui sont d'une dimension telle que la feuille de papier 12 comporte des zones de chute de part et d'autre d'une zone utile de la feuille 12. Éventuellement, la ou les bandes de compensation de charge 24 pourront alors être prévues au moins en partie, dans ces zones de chute.

Enfin, dans le cas où les seconds éléments en relief de la bande de compensation de charge se distinguent des premiers éléments en relief de la bande de serrage, il se peut que pour certains motifs marqués sur le produit fini, les éléments en relief de la bande de compensation de charge 24 présente un caractère suffisamment esthétique pour pouvoir être conservés sur le produit final que constitue l'article en papier. Dans ce cas, on pourra avantageusement prévoir que la bande de compensation de charge 24 soit agencée sur le cylindre 14 de manière à être au moins partiellement en contact avec la feuille de papier 12, ce qui aura pour conséquence que la trace de la bande de compensation de charge 24 sera reproduite au moins partiellement sur la feuille de papier 12 formant ainsi un marquage complémentaire. On pourra choisir pour la bande de compensation de charge des éléments en relief similaires mais non identiques (par exemple de dimension différente) à ceux de la bande de serrage, que l'on adaptera.

En fonction de la disposition des éléments en relief de la bande de serrage 18, il peut s'avérer que la largeur de la bande de compensation de charge 24 nécessaire pour équilibrer l'effort d'interaction des cylindres 14, 16 soit excessive par rapport à la place disponible sur le cylindre 14. Dans ce cas, on peut se contenter d'utiliser une ou plusieurs bandes de compensation partielles qui ne compensent pas entièrement les variations des surfaces actives instantanées successives de la bande de serrage au cours de la rotation des deux cylindres 14 et 16 mais qui permettent malgré tout de les limiter dans des proportions importantes afin d'atteindre l'objectif d'augmentation de la durée de vie des cylindres. Dans ce cas, la variation des surfaces actives instantanées successives correspondant à la bande de serrage et à la bande de compensation de charge est inférieure à la variation des surfaces actives instantanées successives correspondant à la seule bande de serrage 18.

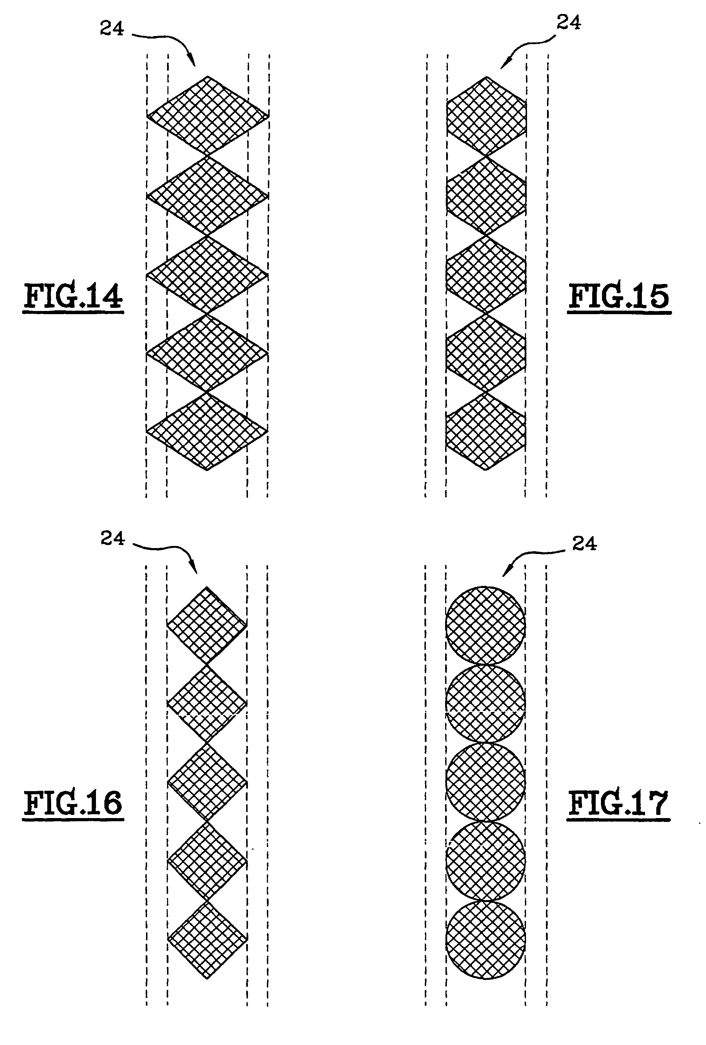

Les figures 15, 16 et 17 illustrent différents modes de définition d'une bande de compensation partielle susceptible d'être utilisée à la place de la bande de compensation théorique représentée à la figure 14 qui est par ailleurs du type délimité par deux bords formés par une ligne brisée.

Dans l'exemple de réalisation de la figure 15, la bande de compensation de charge théorique a été écrêtée, c'est-à-dire que toutes les portions de la bande théorique dont la largeur dépasse une largeur maximale disponible sont ramenées à cette dite largeur maximale.

Dans l'exemple de réalisation de la figure 16, la largeur de la bande de compensation de charge théorique a été réduite d'un facteur constant de manière que la largeur maximale de la bande de compensation partielle ne dépasse pas la largeur maximale disponible. Dans cet exemple de réalisation, on a réduit la bande de compensation de charge en gardant sensiblement la forme de la bande de compensation de charge théorique.

Enfin, on a représenté sur la figure 17 un troisième mode de réalisation d'une bande de compensation partielle dont la forme n'est pas directement issue de celle de la bande théorique mais qui permet malgré tout de diminuer la variation des efforts transmis entre les deux cylindres 14, 16. Suivant ce mode de réalisation, il est possible de favoriser un des paramètres de la bande de compensation de charge : les zones où la surface active de la bande de compensation de charge est nulle, les zones où elle est maximale, les zones de transition, etc. La forme de la bande de compensation de charge peut être obtenue à partir de la forme théorique à l'aide d'une fonction mathématique.

Les deux cylindres du dispositif selon l'invention peuvent être constitués d'un même matériau ou de deux matériaux différents. Au moins l'un des deux est gravé. Les deux peuvent être en matériau non déformable tel que l'acier ou l'un en matériau non déformable et l'autre en matériau déformable par exemple en caoutchouc.

Chacun des cylindres peut être constitué d'un matériau unique ou de plusieurs matériaux.

Dans ce dernier cas, le cylindre gravé est constitué d'une partie centrale pour la gravure des éléments en relief de la bande de serrage, réalisée par exemple en acier, d'une ou plusieurs parties latérales éventuellement réalisées dans un autre matériau pour la bande de compensation de charge et éventuellement, d'une ou plusieurs autres parties disposées latéralement aux bandes de compensation de charge pour la zone de roulement. Ces autres parties peuvent être réalisées dans le matériau des bandes de compensation de charge ou encore dans un autre matériau. Les matériaux pour la bande de compensation de charge et pour les autres parties sont des matériaux non déformables, identiques ou différents. Le contre-cylindre comprend une partie centrale réalisée par exemple en caoutchouc pour le vis à vis des éléments en relief de la bande de serrage du cylindre gravé, d'une ou plusieurs parties latérales pour le vis à vis de la bande de compensation de charge et, le cas échéant, de la zone de roulement. Ces parties latérales venant au contact de la bande de compensation de charge et, le cas échéant, de la zone de roulement, peuvent être réalisées en caoutchouc ou à partir d'un ou plusieurs autres matériaux. Les parties latérales correspondant respectivement à la bande de compensation de charge et à la zone de roulement peuvent être réalisées dans un matériau identique ou différent.

Les cylindres gravé et contrepartie s'ils sont réalisés dans un même matériau, peuvent encore comporter des zones de traitement de surface différentes.

Les procédés mettant en oeuvre les dispositifs selon l'invention peuvent indépendamment conduire à la déformation d'une feuille de papier ou à l'association de plusieurs feuilles de papier ou encore simultanément à la déformation et à l'association de plusieurs feuilles, selon les caractéristiques du procédé et l'installation comportant le ou les dispositifs selon l'invention.

Dans le cas d'une association d'au moins deux feuilles de papier ou nontissé, le procédé met en oeuvre un dispositif selon l'invention dans lequel les feuilles sont serrées entre les deux cylindres au niveau de la bande de serrage, avec ou sans apport d'adhésif.

Dans le cas du marquage strict, un seul dispositif constitué de deux cylindres dont l'un est muni d'une bande de serrage, peut suffire.

Dans le cas du gaufrage, plusieurs solutions sont possibles. Par exemple, deux feuilles peuvent être gaufrées par la mise en oeuvre de deux dispositifs indépendants ou dépendants, puis être associées par la mise en oeuvre d'un troisième dispositif. De préférence, ce troisième dispositif a au moins un cylindre en commun (le cylindre gravé) avec l'un des deux premiers dispositifs mis en oeuvre pour le gaufrage.

Suivant un autre mode de réalisation, toujours dans le cas du gaufrage, les deux premiers dispositifs sont dépendants (ont un cylindre en commun) et assurent chacun le gaufrage d'une feuille ; l'association est réalisée par un dispositif constitué de deux cylindres appartenant chacun respectivement aux premier et second dispositifs.

L'invention vise également un procédé d'application d'un additif tel qu'un adhésif, une lotion ou de l'encre, sur une feuille de papier ou nontissé comportant la mise en oeuvre d'un dispositif selon l'invention.

L'invention permet ainsi d'obtenir des articles en papier ou en nontissé marqués ou gaufrés suivant des motifs très variés. Elle fournit également des produits comportant plusieurs feuilles solidaires ou associées entre elles.

## Revendications

1. Dispositif pour le serrage d'au moins une feuille de papier ou de nontissé (12) comprenant deux cylindres (14, 16) sensiblement tangents l'un à l'autre, au moins un des cylindres comportant sur sa surface dans une zone destinée à être en contact avec ladite feuille (12), une bande de serrage (18) comprenant des premiers éléments en relief, l'interaction des deux cylindres avec ou sans la feuille définissant une surface active au cours de la rotation des cylindres, **caractérisé en ce que** l'un (14) au moins des cylindres comporte sur sa surface dans une zone destinée à être en contact avec l'autre cylindre par l'intermédiaire ou non de la feuille, une bande de compensation de charge (24) comprenant des seconds éléments en relief et choisie de façon que la variation des surfaces actives instantanées successives correspondant à la bande de serrage (18) et à la bande de compensation de charge (24), soit inférieure à la variation des surfaces actives instantanées successives correspondant à la seule bande de serrage (18).

2. Dispositif selon la revendication 1, **caractérisé en ce que** la variation des surfaces actives instantanées successives correspondant à la bande de serrage (18) et à la bande de compensation de charge (24) est sensiblement nulle.

3. Dispositif selon l'une des revendications précédentes, **caractérisé en ce que** la largeur de la surface active correspondant à la seule bande de compensation de charge (24) varie le long de la circonférence du cylindre sur lequel ladite bande a été créée, en fonction de la surface active instantanée correspondant à la seule bande de serrage (18).

4. Dispositif selon l'une des revendications précédentes, **caractérisé en ce que** les cylindres précités sont contrarotatifs.

5. Dispositif selon l'une des revendications précédentes, **caractérisé en ce que** les premiers éléments en relief de la bande de serrage (18) sont identiques ou différents des seconds éléments en relief de la bande de compensation de charge (24).

6. Dispositif selon l'une des revendications précédentes, **caractérisé en ce que** la bande de compensation de charge (24) est agencée sur un cylindre (14) en dehors de ladite bande de serrage (18).

7. Dispositif selon l'une des revendications 1 à 5, **caractérisé en ce que** les seconds éléments en relief de la bande de compensation de charge sont différents des premiers éléments en relief de la bande de serrage et **en ce que** la bande de compensation de charge (24) chevauche au moins partiellement la bande de serrage .

8. Dispositif selon l'une quelconque des revendications précédentes, **caractérisé en ce qu'**au moins un (14) des cylindres porte au moins une bande de serrage (18) et au moins une bande de compensation de charge (24).

9. Dispositif selon la revendication 8, **caractérisé en ce que** la hauteur des éléments en relief de la bande de compensation de charge (24) est sensiblement identique à la hauteur des éléments en relief de la bande de serrage (18).

10. Dispositif selon la revendication 8, **caractérisé en ce que** la hauteur des éléments en relief de la bande de compensation de charge (24) est supérieure à la hauteur des éléments en relief de la bande de serrage (18).

11. Dispositif selon la revendication 8, **caractérisé en ce que** la hauteur des éléments en relief de la bande de compensation de charge (24) est inférieure à la hauteur des éléments en relief de la bande de serrage (18).

12. Dispositif selon l'une quelconque des revendications précédentes, **caractérisé en ce que** la bande de compensation de charge (24) comporte une zone de roulement (26) qui détermine une largeur minimale de la surface active de la bande de compensation de charge (24).

13. Dispositif selon la revendication 12, **caractérisé en ce que** la zone de roulement (26) de la bande de compensation de charge (24) est agencée radialement au même niveau que le reste de la bande de compensation de charge (24).

14. Dispositif selon la revendication 12, **caractérisé en ce que** la zone de roulement (26) de la bande de compensation de charge (24) est agencée radialement vers l'extérieur par rapport au reste de la bande de compensation de charge (24).

15. Dispositif selon la revendication 12, **caractérisé en ce que** la zone de roulement (26) de la bande de compensation de charge (24) est agencée radialement vers l'intérieur par rapport au reste de la bande de compensation de charge (24).

16. Dispositif selon l'une quelconque des revendications précédentes, **caractérisé en ce que** plusieurs bandes de compensation de charge (24) sont prévues sur le même cylindre.

17. Dispositif selon la revendication 16 prise en combinaison avec l'une quelconque des revendications 12 à 15, **caractérisé en ce que** chacune des bandes de compensation de charge (24) comporte une zone de roulement (26).

18. Dispositif selon l'une des revendications précédentes, **caractérisé en ce que** les deux cylindres sont en matériau non déformable.

19. Dispositif selon l'une des revendications 1 à 17, **caractérisé en ce que** l'un des cylindres est en matériau non déformable et l'autre est en matériau déformable.

20. Cylindre pour le serrage d'une feuille de papier ou nontissé, **caractérisé en ce qu'**il fait partie du dispositif selon l'une quelconque des revendications précédentes et **en ce qu'**il comporte une bande de compensation de charge (24).

21. Installation comprenant au moins un dispositif de serrage selon l'une des revendications 1 à 19.

22. Installation selon la revendication 21, **caractérisée en ce qu'**elle comprend au moins deux dispositifs selon l'une des revendications 1 à 19, lesdits dispositifs étant indépendants l'un de l'autre.

23. Installation selon la revendication 21, **caractérisée en ce qu'**elle comprend au moins deux dispositifs selon l'une des revendications 1 à 19, au moins deux desdits dispositifs ayant un cylindre en commun.

24. Procédé d'association d'au moins deux feuilles de papier ou nontissé, **caractérisé en ce qu'**il comporte la mise en oeuvre d'un dispositif selon l'une quelconque des revendications 1 à 19, dans lequel lesdites feuilles sont serrées entre les deux cylindres au niveau de la bande de serrage (18), avec ou sans apport d'adhésif.

25. Procédé de marquage d'au moins une feuille de papier ou nontissé, **caractérisé en ce qu'**il comporte la mise en oeuvre d'un dispositif selon l'une quelconque des revendications 1 à 19, dans lequel la bande de serrage (18) réalise sur le papier ou nontissé un motif par déformation de la feuille qui est serrée entre les deux cylindres.

26. Procédé selon la revendication 25, **caractérisé en ce qu'**il s'agit d'un gaufrage.

27. Procédé selon la revendication 26, **caractérisé en ce que** deux feuilles de papier ou nontissé sont gaufrées par la mise en oeuvre respectivement de deux dispositifs indépendants ou dépendants selon l'une quelconque des revendications 1 à 19, puis associées par la mise en oeuvre d'un troisième dispositif selon l'une quelconque des revendications 1 à 19.

28. Procédé selon la revendication 27, **caractérisé en ce que** le troisième dispositif a au moins un cylindre gravé en commun avec l'un des deux dispositifs précités mis en oeuvre pour le gaufrage.

29. Procédé d'application d'un additif tel qu'un adhésif, une lotion ou de l'encre, sur une feuille de papier ou nontissé, **caractérisé en ce qu'**il comporte la mise en oeuvre d'un dispositif selon l'une des revendications 1 à 19.

30. Procédé de fabrication d'articles en ouate de cellulose comportant au moins deux feuilles, **caractérisé en ce que** les feuilles sont rendues solidaires les unes aux autres par un procédé de marquage, gaufrage ou association selon l'une des revendications 24 à 28.

## Patentansprüche

1. Vorrichtung zum Prägen von mindestens einem Blatt Papier oder Vliesstoff (12), die zwei Walzen (14, 16) umfasst, die einander beinahe berühren, wobei mindestens eine Walze auf ihrer Oberfläche, in einem Bereich, der dafür gedacht ist, mit diesem Blatt (12) in Berührung zu stehen, ein Prägeband (18) umfasst, das erste hervorstehende Bestandteile aufweist, wobei durch das Wechselwirken der zwei Walzen mit oder ohne Blatt während des Drehens der Walzen eine wirksame Fläche beschrieben ist, **dadurch gekennzeichnet, dass** mindestens eine (14) der Walzen auf ihrer Oberfläche, in einem Bereich, der dafür gedacht ist, mit der anderen Walze über das Blatt oder ohne das Blatt in Berührung zu stehen, ein. Belastungsausgleichsband (24) umfasst, das zweite hervorstehende Bestandteile aufweist und so gewählt ist, dass die Veränderung der aufeinander folgenden momentanen wirksamen Flächen, die dem Prägeband (18) und dem Belastungsausgleichsband (24) entsprechen, geringer ist als die Veränderung der aufeinander folgenden momentanen wirksamen Flächen, die nur dem Prägeband (18) entsprechen.

2. Vorrichtung nach Anspruch 1, **dadurch gekennzeichnet, dass** die Veränderung der aufeinander folgenden momentanen wirksamen Flächen, die dem Prägeband (18) und dem Belastungsausgleichsband (24) entsprechen, ungefähr null ist.

3. Vorrichtung nach einem der vorhergehenden Ansprüche, **dadurch gekennzeichnet, dass** sich die Breite der wirksamen Fläche, die nur dem Belastungsausgleichsband (24) entspricht, entlang dem Umfang der Walze ändert, auf der dieses Band erzeugt wurde, in Abhängigkeit von der wirksamen Fläche, die nur dem Prägeband (18) entspricht.

4. Vorrichtung nach einem der vorhergehenden Ansprüche, **dadurch gekennzeichnet, dass** die zuvor genannten Walzen gegenläufig sind.

5. Vorrichtung nach einem der vorhergehenden Ansprüche, **dadurch gekennzeichnet, dass** die ersten hervorstehenden Bestandteile des Prägebands (18) vollkommen übereinstimmen mit oder verschieden sind von den zweiten hervorstehenden Bestandteilen des Belastungsausgleichsbands (24).

6. Vorrichtung nach einem der vorhergehenden Ansprüche, **dadurch gekennzeichnet, dass** das Belastungsausgleichsband (24) auf einer Walze (14) außerhalb des Prägebands (18) angeordnet ist.

7. Vorrichtung nach einem der Ansprüche 1 bis 5, **dadurch gekennzeichnet, dass** die zweiten hervorstehenden Bestandteile des Belastungsausgleichsbands verschieden sind von den ersten hervorstehenden Bestandteilen des Prägebands und **dadurch gekennzeichnet, dass** sich das Belastungsausgleichsband (24) zumindest teilweise mit dem Prägeband überschneidet.

8. Vorrichtung nach einem der vorhergehenden Ansprüche, **dadurch gekennzeichnet, dass** sich auf mindestens einer Walze (14) mindestens ein Prägeband (18) und mindestens ein Belastungsausgleichsband (24) befindet.

9. Vorrichtung nach Anspruch 8, **dadurch gekennzeichnet, dass** die Höhe der hervorstehenden Bestandteile des Belastungsausgleichsbands (24) ungefähr der Höhe der hervorstehenden Bestandteile des Prägebands (18) entspricht.

10. Vorrichtung nach Anspruch 8, **dadurch gekennzeichnet, dass** die Höhe der hervorstehenden Bestandteile des Belastungsausgleichsbands (24) größer ist als die Höhe der hervorstehenden Bestandteile des Prägebands (18).

11. Vorrichtung nach Anspruch 8, **dadurch gekennzeichnet, dass** die Höhe der hervorstehenden Bestandteile des Belastungsausgleichsbands (24) geringer ist als die Höhe der hervorstehenden Bestandteile des Prägebands (18).

12. Vorrichtung nach einem der vorhergehenden Ansprüche, **dadurch gekennzeichnet, dass** das Ausgleichsband (24) einen Laufbereich (26) umfasst, der eine minimale Breite der wirksamen Fläche des Belastungsausgleichsbands (24) bestimmt.

13. Vorrichtung nach Anspruch 12, **dadurch gekennzeichnet, dass** der Laufbereich (26) des Belastungsausgleichsbands (24) vom Mittelpunkt ausgehend auf gleicher Höhe wie das übrige Belastungsausgleichsband (24) angeordnet ist.

14. Vorrichtung nach Anspruch 12, **dadurch gekennzeichnet, dass** der Laufbereich (26) des Ausgleichsbands (24) vom Mittelpunkt ausgehend nach außen im Verhältnis zum übrigen Belastungsausgleichsband (24) angeordnet ist.

15. Vorrichtung nach Anspruch 12, **dadurch gekennzeichnet, dass** der Laufbereich (26) des Belastungsausgleichsbands (24) vom Mittelpunkt ausgehend nach innen im Verhältnis zum übrigen Belastungsausgleichsband (24) angeordnet ist.

16. Vorrichtung nach einem der vorhergehenden Ansprüche, **dadurch gekennzeichnet, dass** auf der gleichen Walze mehrere Belastungsausgleichsbänder (24) vorgesehen sind.

17. Vorrichtung nach Anspruch 16 in Verbindung mit einem der Ansprüche 12 bis 15, **dadurch gekennzeichnet, dass** jedes Belastungsausgleichsband (24) einen Laufbereich (26) umfasst.

18. Vorrichtung nach einem der vorhergehenden Ansprüche, **dadurch gekennzeichnet, dass** beide Walzen aus einem nicht verformbaren Werkstoff bestehen.

19. Vorrichtung nach einem der Ansprüche 1 bis 17, **dadurch gekennzeichnet, dass** eine Walze aus einem nicht verformbaren Werkstoff und die andere aus einem verformbaren Werkstoff besteht.

20. Walze zum Prägen von einem Blatt Papier oder Vliesstoff, **dadurch gekennzeichnet, dass** sie Teil der Vorrichtung nach einem der vorhergehenden Ansprüche ist und **dadurch gekennzeichnet, dass** sie ein Belastungsausgleichsband (24) umfasst.

21. Einrichtung, die mindestens eine Vorrichtung zum Prägen nach einem der Ansprüche 1 bis 19 umfasst.

22. Einrichtung nach Anspruch 21, **dadurch gekennzeichnet, dass** sie mindestens zwei Vorrichtungen nach einem der Ansprüche 1 bis 19 umfasst, wobei die Vorrichtungen unabhängig voneinander sind.

23. Einrichtung nach Anspruch 21, **dadurch gekennzeichnet, dass** sie mindestens zwei Vorrichtungen nach einem der Ansprüche 1 bis 19 umfasst, wobei mindestens zwei der Vorrichtungen eine gemeinsame Walze aufweisen.

24. Verfahren zum Verbinden von mindestens zwei Blättern Papier oder Vliesstoff, **dadurch gekennzeichnet, dass** es den Einsatz einer Vorrichtung nach einem der Ansprüche 1 bis 19 umfasst, in der die Blätter zwischen den beiden Walzen auf Höhe des Prägebands (18) geprägt werden, mit oder ohne Zugabe von Klebstoff.

25. Verfahren zum Kennzeichnen von mindestens einem Blatt Papier oder Vliesstoff, **dadurch gekennzeichnet, dass** es den Einsatz einer Vorrichtung nach einem der Ansprüche 1 bis 19 umfasst, in dem das Prägeband (18) auf dem Papier oder Vliesstoff ein Muster durch Verformen des Blatts erzeugt, das zwischen den beiden Walzen geprägt wird.

26. Verfahren nach Anspruch 25, **dadurch gekennzeichnet, dass** es sich um eine Gaufrage handelt.

27. Verfahren nach Anspruch 26, **dadurch gekennzeichnet, dass** zwei Blätter Papier oder Vliesstoff von zwei eigenständigen bzw. abhängigen Vorrichtungen nach einem der Ansprüche 1 bis 19 gaufriert und dann durch den Einsatz einer dritten Vorrichtung nach einem der Ansprüche 1 bis 19 verbunden sind.

28. Verfahren nach Anspruch 27, **dadurch gekennzeichnet, dass** die dritte Vorrichtung mindestens eine gravierte Walze aufweist, die sie mit einer der beiden zuvor genannten Vorrichtungen gemein hat, die für die Gaufrage eingesetzt werden.

29. Verfahren zum Aufbringen eines Zusatzstoffs wie einem Klebstoff, einer Lotion oder Tinte auf einem Blatt Papier oder Vliesstoff, **dadurch gekennzeichnet, dass** es den Einsatz einer Vorrichtung nach einem der Ansprüche 1 bis 19 umfasst.

30. Verfahren zum Herstellen von Produkten aus Zellstoffwatte, die mindestens zwei Blätter umfassen, **dadurch gekennzeichnet, dass** die Blätter fest miteinander verbunden werden durch ein Kennzeichnungs-, Gaufrageoder Verbindungsverfahren nach einem der Ansprüche 24 bis 28.

## Claims

1. Device for clamping at least one paper or non-woven sheet (12) comprising two cylinders (14, 16) substantially tangent to each other, at least one of the cylinders having on its surface, in a zone intended to be in contact with the said sheet (12), a clamping band (18) comprising first salient elements, the interaction of the two cylinders with or without the sheet defining an active surface during the rotation of the cylinders, **characterised in that** at least one (14) of the cylinders has on its surface, in a zone intended to be in contact with the other cylinder, through the intermediary or not of the sheet, a load compensating band (24) comprising second salient elements and chosen so that the variation of the successive instantaneous active surfaces corresponding to the clamping band (18) and to the load compensating band (24) is less than the variation of the successive instantaneous active surfaces corresponding to the clamping band (18) alone.

2. Device according to Claim 1, **characterised in that** the variation of the successive instantaneous active surfaces corresponding to the clamping band (18) and to the load compensating band (24) is substantially zero.

3. Device according to one of the preceding claims, **characterised in that** the width of the active surface corresponding to the load compensating band (24) alone varies along the circumference of the cylinder on which the said band has been created, as a function of the instantaneous active surface corresponding to the clamping band (18) alone.

4. Device according to one of the preceding claims, **characterised in that** the aforementioned cylinders rotate in opposite directions.

5. Device according to one of the preceding claims, **characterised in that** the first salient elements of the clamping band (18) are identical to or different from the second salient elements of the load compensating band (24).

6. Device according to one of the preceding claims, **characterised in that** the load compensating band (24) is arranged on a cylinder (14) outside the said clamping band (18).

7. Device according to one of Claims 1 to 5, **characterised in that** the second salient elements of the load compensating band differ from the first salient elements of the clamping band and **in that** the load compensating band (24) at least partly overlaps the clamping band.

8. Device according to any one of the preceding claims, **characterised in that** at least one (14) of the cylinders bears at least one clamping band (18) and at least one load compensating band (24).

9. Device according to Claim 8, **characterised in that** the height of the salient elements of the load compensating band (24) is substantially identical to the height of the salient elements of the clamping band (18).

10. Device according to Claim 8, **characterised in that** the height of the salient elements of the load compensating band (24) is greater than the height of the salient elements of the clamping band (18).

11. Device according to Claim 8, **characterised in that** the height of the salient elements of the load compensating band (24) is less than the height of the salient elements of the clamping band (18).

12. Device according to any one of the preceding claims, **characterised in that** the load compensating band (24) has a rolling zone (26) which determines a minimum width of the active surface of the load compensating band (24).

13. Device according to Claim 12, **characterised in that** the rolling zone (26) of the load compensating band (24) is arranged radially at the same level as the remainder of the load compensating band (24).

14. Device according to Claim 12, **characterised in that** the rolling zone (26) of the load compensating band (24) is arranged radially outwards relative to the remainder of the load compensating band (24).

15. Device according to Claim 12, **characterised in that** the rolling zone (26) of the load compensating band (24) is arranged radially inwards relative to the remainder of the load compensating band (24).

16. Device according to any one of the preceding claims, **characterised in that** a plurality of load compensating bands (24) are provided on the same cylinder.

17. Device according to Claim 16, taken in combination with any one of Claims 12 to 15, **characterised in that** each of the load compensating bands (24) has a rolling zone (26).

18. Device according to one of the preceding claims, **characterised in that** the two cylinders are made of non-deformable material.

19. Device according to one of Claims 1 to 17, **characterised in that** one of the cylinders is made of non-deformable material and the other is made of deformable material.

20. Cylinder for clamping a paper or non-woven sheet, **characterised in that** it forms part of the device according to any one of the preceding claims and **in that** it has a load compensating band (24).

21. Installation comprising at least one clamping device according to one of Claims 1 to 19.

22. Installation according to Claim 21, **characterised in that** it comprises at least two devices according to one of Claims 1 to 19, the said devices being independent of one another.

23. Installation according to Claim 21, **characterised in that** it comprises at least two devices according to one of Claims 1 to 19, at least two of the said devices sharing a cylinder.

24. Method for associating at least two paper or non-woven sheets, **characterised in that** it involves employing a device according to any one of Claims 1 to 19, in which the said sheets are clamped between the two cylinders at the clamping band (18), with or without adhesive being supplied.

25. Method for marking at least one paper or non-woven sheet, **characterised in that** it involves employing a device according to any one of Claims 1 to 19, in which the clamping band (18) produces a pattern on the paper or non-woven by deforming the sheet which is clamped between the two cylinders.

26. Method according to Claim 25, **characterised in that** it involves embossing.

27. Method according to Claim 26, **characterised in that** two paper or non-woven sheets are embossed by respectively employing two independent or dependent devices according to any one of Claims 1 to 19 and are then associated by employing a third device according to any one of Claims 1 to 19.

28. Method according to Claim 27, **characterised in that** the third device shares at least one engraved cylinder with one of the two aforementioned devices employed for the embossing.

29. Method for applying an additive, such as an adhesive, a lotion or ink, to a paper or non-woven sheet, **characterised in that** it involves employing a device according to one of Claims 1 to 19.

30. Method for manufacturing cellulose wadding articles having at least two sheets, **characterised in that** the sheets are joined to one another by a marking, embossing or associating method according to one of Claims 24 to 28.
